# EUROPEAN PATENT APPLICATION

(11) **EP 3 488 788 A1**
(43) Date of publication of application: **29.05.2019**
(21) Application number: 17830857.3
(22) Date of filing: 06.07.2017
(51) Int. Cl.: A61B 8/08, A61B 8/12, A61B 8/14

(54) **ULTRASONIC OBSERVATION DEVICE, OPERATION METHOD OF ULTRASONIC OBSERVATION DEVICE, AND OPERATION PROGRAM OF ULTRASONIC OBSERVATION DEVICE**

(30) Priority: 19.07.2016 JP 2016141618
(71) Applicant: Olympus Corporation, Hachioji-shi Tokyo 192-8507 (JP)
(72) Inventor: MIYAKE, Tatsuya, Hachioji-shi Tokyo 192-8507 (JP)
(74) Representative: Gunzelmann, Rainer
(86) International application number: PCT/JP2017/024798
(87) International publication number: WO 2018/016337

(57) **Abstract**

An ultrasonic observation apparatus including: an image processing unit to create an ultrasonic image based on an ultrasonic signal received from an ultrasonic transducer that transmits an ultrasonic wave to an observation target and receives an ultrasonic wave reflected from the observation target; an elasticity information calculation unit to calculate elasticity information of the observation target in a predetermined region within the ultrasonic image; a region extraction unit to extract, from the predetermined region, a region where the elasticity information satisfies a predetermined condition; a calculation unit to calculate diagnosis support information that supports an operator to determine a diagnosis sequence of each extracted region on the basis of the elasticity information of the corresponding region; and an image synthesizing unit to create an image by synthesizing the diagnosis support information into the ultrasonic image. It is possible to provide an ultrasonic observation apparatus capable of extracting a region to be preferentially diagnosed without consuming time or labor of the operator.

## Description

### Field

The present invention relates to an ultrasonic observation apparatus, a method of operating the ultrasonic observation apparatus, and a program for operating the ultrasonic observation apparatus.

### Background

In the related art, there is known "ultrasonic elastography" as a technique of diagnosing an observation target using ultrasonic waves (for example, see Patent Literature 1). Ultrasonic elastography is a technique that utilizes a fact that a cancer or tumor tissue in a living organism has different stiffness depending on a disease progress status or a living organism. In this technique, coloring is performed by setting an average value of a displacement of a biological tissue in a predetermined region of interest (ROI) as a reference value, so as to create an elasticity image by imaging information regarding stiffness of the biological tissue. In the ultrasonic elastography, an operator such as a doctor sets the region of interest depending on details of the observation.

### Citation List

### Patent Literature

Patent Literature 1: JP 2009-261686 A

### Summary

### Technical Problem

However, in the ultrasonic elastography of the related art, an operator observes an elasticity image of the entire ultrasonic image by setting the region of interest to the entire ultrasonic image to search a region to be diagnosed preferentially out of high stiffness regions considered as cancer or tumor. This consumes time or labor.

In view of the aforementioned problems, an object of the invention is to provide an ultrasonic observation apparatus, a method of operating the ultrasonic observation apparatus, and a program for operating the ultrasonic observation apparatus, capable of extracting a region to be preferentially diagnosed without consuming time or labor of an operator.

### Solution to Problem

To solve the above-described problem and to accomplish the above-described object, according to the present invention, there is provided an ultrasonic observation apparatus comprising: an image processing unit configured to create an ultrasonic image based on an ultrasonic signal received from an ultrasonic transducer that transmits an ultrasonic wave to an observation target and receives an ultrasonic wave reflected from the observation target; an elasticity information calculation unit configured to calculate elasticity information of the observation target in a predetermined region within the ultrasonic image; a region extraction unit configured to extract a region where the elasticity information calculated by the elasticity information calculation unit in the predetermined region satisfies a predetermined condition; a calculation unit configured to calculate diagnosis support information that supports an operator to determine a diagnosis sequence of each region extracted by the region extraction unit on the basis of the elasticity information of the corresponding region; and an image synthesizing unit configured to create an image by synthesizing the diagnosis support information calculated by the calculation unit into the ultrasonic image.

In the ultrasonic observation apparatus according to the present invention, the predetermined condition on the elasticity information includes a condition that stiffness is equal to or higher than a predetermined threshold value, a condition that the stiffness equal to or higher than the threshold value is maintained for a predetermined period of time or longer, or a condition that a region of the stiffness equal to or higher than the threshold value occupies a predetermined area or larger.

In the ultrasonic observation apparatus according to the present invention, the diagnosis support information is a priority for diagnosing each region extracted by the region extraction unit, the priority being determined by the calculation unit based on the elasticity information.

The ultrasonic observation apparatus according to the present invention further comprises a region-of-interest setting unit configured to set, as a region of interest, a region including the region extracted by the region extraction unit, wherein the image synthesizing unit creates an image by synthesizing elastographic image data of the region of interest set by the region-of-interest setting unit into the ultrasonic image.

The ultrasonic observation apparatus according to the present invention further comprises an input unit configured to receive an input of an operator, wherein the region-of-interest setting unit switches, in response to an input received by the input unit, the region of interest sequentially from the region including the high priority region calculated by the calculation unit.

In the ultrasonic observation apparatus according to the present invention, the region-of-interest setting unit sets the region of interest by centering a center of mass of the region extracted by the region extraction unit such that a ratio between an area of the extracted region and an area of a surrounding region thereof becomes a predetermined value.

In the ultrasonic observation apparatus according to the present invention, the region extraction unit extracts, based on the elasticity information calculated by the elasticity information calculation unit, a closed region where a relatively stiff region occupies a predetermined area or larger for a predetermined period of time or longer.

In the ultrasonic observation apparatus according to the present invention, the calculation unit sets, as the priority, a descending order of higher stiffness based on the elasticity information of each region extracted by the region extraction unit.

In the ultrasonic observation apparatus according to the present invention, the calculation unit sets, as the priority, a descending order of a larger area of each region extracted by the region extraction unit.

In the ultrasonic observation apparatus according to the present invention, the image synthesizing unit creates an image by synthesizing each region extracted by the region extraction unit into the ultrasonic image such that interference with the ultrasonic image is reduced.

In the ultrasonic observation apparatus according to the present invention, the image synthesizing unit creates an image by synthesizing each region extracted by the region extraction unit such that each extracted region is identified with a dashed line, a dotted line, or a solid line.

In the ultrasonic observation apparatus according to the present invention, the image synthesizing unit creates an image by synthesizing the priority calculated by the calculation unit such that the priority is identified with a numerical value or a color.

According to the present invention, there is provided a method of operating an ultrasonic observation apparatus that creates an ultrasonic image based on an ultrasonic signal received from an ultrasonic transducer that transmits an ultrasonic wave to an observation target and receives an ultrasonic wave reflected from the observation target, the method comprising: an elasticity information calculation step of calculating elasticity information of the observation target in a predetermined region within the ultrasonic image using an elasticity information calculation unit; a region extraction step of extracting a region where the elasticity information calculated by the elasticity information calculation unit in the predetermined region satisfies a predetermined condition using a region extraction unit; a calculation step of calculating diagnosis support information that supports an operator to determine a diagnosis sequence of each region on the basis of the elasticity information of each region extracted by the region extraction unit using a calculation unit; and an image synthesizing step of creating an image by synthesizing the diagnosis support information calculated by the calculation unit into the ultrasonic image using an image synthesizing unit.

According to the present invention, there is provided a program for operating an ultrasonic observation apparatus that creates an ultrasonic image based on an ultrasonic signal received from an ultrasonic transducer that transmits an ultrasonic wave to an observation target and receives an ultrasonic wave reflected from the observation target, the program causes the ultrasonic observation apparatus to execute steps comprising: an elasticity information calculation step of calculating elasticity information of the observation target in a predetermined region within the ultrasonic image using an elasticity information calculation unit; a region extraction step of extracting a region where the elasticity information calculated by the elasticity information calculation unit in the predetermined region satisfies a predetermined condition using a region extraction unit; a calculation step of calculating diagnosis support information that supports an operator to determine a diagnosis sequence of each region on the basis of the elasticity information of each region extracted by the region extraction unit using a calculation unit; and an image synthesizing step of creating an image by synthesizing the diagnosis support information calculated by the calculation unit into the ultrasonic image using an image synthesizing unit.

### Advantageous Effects of Invention

According to the invention, it is possible to implement an ultrasonic observation apparatus, a method of operating the ultrasonic observation apparatus, and a program for operating the ultrasonic observation apparatus, capable of extracting a region to be preferentially diagnosed without consuming time or labor of an operator.

### Brief Description of Drawings

FIG. 1 is a diagram schematically illustrating a configuration of an ultrasonic diagnosis system having an ultrasonic observation apparatus according to an embodiment of the invention.
FIG. 2 is a flowchart illustrating an outline of a processing of the ultrasonic observation apparatus according to an embodiment of the invention.
FIG. 3 is a diagram illustrating an exemplary image displayed on a display device.
FIG. 4 is a diagram illustrating a state in which a region including a highest priority region is set as a region of interest.
FIG. 5 is a diagram illustrating a state in which a region including a second highest priority region is set as a region of interest.
FIG. 6 is a diagram illustrating a state in which a region including a third highest priority region is set as a region of interest.
FIG. 7 is a diagram illustrating an exemplary image displayed on the display device in an ultrasonic diagnosis system having an ultrasonic observation apparatus according to a modification of the invention.

### Description of Embodiments

Hereinafter, an ultrasonic observation apparatus, a method of operating the ultrasonic observation apparatus, and a program for operating the ultrasonic observation apparatus according embodiments of the invention will be described with reference to the accompanying drawings. Note that the invention is not limited by such embodiments. The invention can generally apply to an ultrasonic observation apparatus capable of diagnosis based on ultrasonic elastography.

In addition, in illustration of drawings, the same or corresponding elements are appropriately denoted by the same reference numerals. Note that the drawings are schematic, and a dimensional relationship, magnification between each element, or the like may be different from those of reality. Portions having different dimensional relationships or magnifications may be included between the drawings.

### (Embodiments)

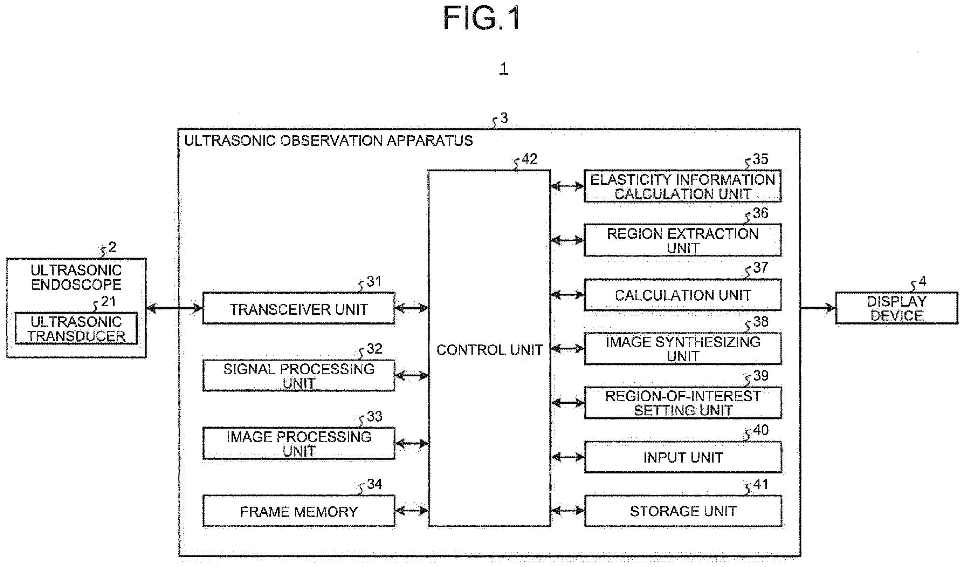

FIG. 1 is a diagram schematically illustrating a configuration of an ultrasonic diagnosis system having an ultrasonic observation apparatus according to an embodiment of the invention. An ultrasonic diagnosis system 1 of FIG. 1 includes an ultrasonic endoscope 2 that transmits ultrasonic waves to a subject as an observation target and receives ultrasonic waves reflected from the subject, an ultrasonic observation apparatus 3 that creates an ultrasonic image on the basis of an ultrasonic signal obtained by the ultrasonic endoscope 2, and a display device 4 that displays the ultrasonic image created by the ultrasonic observation apparatus 3.

The ultrasonic endoscope 2 has, in its distal end portion, an ultrasonic transducer 21 that converts an electric pulse signal received from the ultrasonic observation apparatus 3 into an ultrasonic pulse (acoustic pulse), irradiates the subject with the ultrasonic pulse, converts ultrasonic echo reflected from the subject into an electric echo signal (ultrasonic signal) that expresses a voltage change, and outputs the ultrasonic signal. The ultrasonic transducer 21 includes a convex type transducer. However, the ultrasonic transducer 21 may include a transducer such as radial type or linear type. The ultrasonic endoscope 2 may allow the ultrasonic transducer 21 to perform mechanical scanning, or the ultrasonic transducer 21 may include a plurality of elements provided in an array shape to perform electronic scanning by electronically switching an element relating to transmit/receive operations or delaying transmit/receive operations of each element.

The ultrasonic endoscope 2 typically has an imaging optical system and an image sensor and is inserted into a digestive tract (such as esophagus, stomach, duodenum, and large intestine) or a respiratory organ (such as trachea or bronchus) of the subject to photograph the digestive tract, respiratory organs, or surrounding organs (such as pancreas, gallbladder, bile duct, biliary tract, lymph node, mediastinum, or blood vessels). In addition, the ultrasonic endoscope 2 has a light guide that guides illumination light with which the subject is irradiated during photographing. The light guide has a distal end portion reaching a tip of an insertion portion of the ultrasonic endoscope 2 inserted into the subject and a basal end portion coupled to a light source device that generates the illumination light.

The ultrasonic observation apparatus 3 has a transceiver unit 31, a signal processing unit 32, an image processing unit 33, a frame memory 34, an elasticity information calculation unit 35, a region extraction unit 36, a calculation unit 37, an image synthesizing unit 38, a region-of-interest setting unit 39, an input unit 40, a storage unit 41, and a control unit 42.

The transceiver unit 31 is electrically connected to the ultrasonic endoscope 2 to transmit a transmit signal (pulse signal) of high voltage pulses to the ultrasonic transducer 21 on the basis of a predetermined waveform and a predetermined transmission timing, and receive an echo signal as an electric receive signal from the ultrasonic transducer 21 . In addition, the transceiver unit 31 creates digital radio-frequency (RF) signal data (hereinafter, referred to as "RF data") and outputs it to the signal processing unit 32.

A frequency band of the pulse signal transmitted from the transceiver unit 31 may be set to a wide range that substantially covers a linear response frequency band of electroacoustic conversion from a pulse signal of the ultrasonic transducer 21 to an ultrasonic pulse.

The transceiver unit 31 transmits various control signals output from the control unit 42 to the ultrasonic endoscope 2, and also has functions of receiving various types of information including an identification ID from the ultrasonic endoscope 2 and transmitting various types of information to the control unit 42.

In addition, as the control information for performing elastography is obtained from the control unit 42, the transceiver unit 31 transmits a transmit signal (pulse signal) of high voltage pulses to the ultrasonic transducer 21 on the basis of a waveform and a transmission timing for obtaining a B-mode image and an elastographic image. Specifically, the transceiver unit 31 superimposes the elastographic pulse, for example, on a pulse for acquiring the B-mode image. The transceiver unit 31 transmits ultrasonic waves several times in the same direction and receives a plurality of reflected echo signals to acquire the elastographic echo signal. As the electrographic echo signal is received, the transceiver unit 31 creates elastographic RF data and outputs it to the signal processing unit 32.

The signal processing unit 32 creates digital B-mode receive data on the basis of the RF data received from the transceiver unit 31. Specifically, the signal processing unit 32 creates the digital B-mode receive data by applying a processing known in the art, such as bandpass filtering, envelope demodulation, and logarithmic conversion, to the RF data. In the logarithmic conversion, a common logarithm of the RF data divided by a reference voltage is calculated and expressed as a decibel value. The B-mode receive data includes a plurality of line data in which an amplitude or intensity of the receive signal indicating an intensity of reflection of the ultrasonic pulse is arranged along a transmit/receive direction (depth direction) of the ultrasonic pulse. The signal processing unit 32 outputs the created B-mode receive data for one frame to the image processing unit 33.

In addition, the signal processing unit 32 creates elastographic receive data on the basis of the elastographic RF data received from the transceiver unit 31. Specifically, the signal processing unit 32 calculates a change of the amplitude or intensity of the receive signal indicating an intensity of reflection of the ultrasonic pulse using the codirectional RF data for each predetermined depth, and creates an acoustic ray (line data) corresponding to the calculated change amount. The elastographic receive data includes a plurality of line data in which the change amount in the amplitude or intensity of the receive signal indicating the intensity of reflection of the ultrasonic pulse is arranged along the transmit/receive direction (depth direction) of the ultrasonic pulse. The signal processing unit 32 includes a central processing unit (CPU), various operation circuits, or the like.

The image processing unit 33 creates B-mode image data on the basis of the B-mode receive data received from the signal processing unit 32. The image processing unit 33 creates the B-mode image data by performing a signal processing using techniques known in the art, such as a scan conversion processing, a gain processing, and a contrast processing, and performing data thinning or the like depending on a data step width defined by a display range of the image in the display device 4 to the B-mode receive data output from the signal processing unit 32. In the scan conversion processing, a scan direction of the B-mode receive data is converted from an ultrasonic wave scan direction into a display direction of the display device 4. The ultrasonic image as a B-mode image is a grayscale image in which red (R), green (G), and blue (B) values as variables in an RGB color space match each other. Note that the image created by the image processing unit 33 is larger than a display region displayable by the display device 4. In other words, the B-mode image displayed by the display device 4 is a part of the B-mode image created by the image processing unit 33.

In addition, the image processing unit 33 creates elastographic image data within a region of interest (ROI) set by the region-of-interest setting unit 39 described below on the basis of elasticity information calculated by the elasticity information calculation unit 35 described below. Specifically, the image processing unit 33 creates the elastographic image data by giving color information to each depth position depending on a relative change amount in the set region of interest. The color information is elasticity information representing stiffness of the observation target in each position and is information expressed by a color determined relatively depending on a ratio of the change amount in the region of interest.

The image processing unit 33 creates the B-mode image data and the elastographic image data by performing coordinate transformation in which the B-mode receive data from the signal processing unit 32 and the elasticity information from the elasticity information calculation unit 35 are rearranged to suitably spatially express a scan range and then applying interpolation processing between the B-mode receive data and between the elastographic receive data to fill a gap between the B-mode receive data. The image processing unit 33 includes a CPU, various operation circuits, or the like.

The frame memory 34 includes, for example, a ring buffer to store a single frame of the B-mode image data created by the image processing unit 33 in a time-series manner. The frame memory 34 may also store a plurality of frames of the B-mode image data in a time-series manner. In this case, if the frame memory 34 has an insufficient capacity (stores the predetermined number of frames of the B-mode image data), the predetermined number of frames of the latest B-mode image data are stored in a time-series manner by overwriting the oldest B-mode image data with the latest B-mode image data.

The elasticity information calculation unit 35 calculates elasticity information of the observation target in a predetermined region within the ultrasonic image on the basis of the elastographic receive data received from the signal processing unit 32. The predetermined region is, for example, the entire ultrasonic image, and the elasticity information calculation unit 35 calculates elasticity information in each position within the ultrasonic image. However, the predetermined region may include, for example, a predetermined region or the like located in the center of the ultrasonic image, without limiting to the entire ultrasonic image. Here, the elasticity information refers to, for example, a modulus of elasticity, a displacement, or the like.

The region extraction unit 36 extracts, from the ultrasonic image, a region where the elasticity information of each position calculated by the elasticity information calculation unit 35 satisfies a predetermined condition. Here, the predetermined condition may include, for example, a condition that stiffness is equal to or higher than a predetermined threshold value, a condition that the stiffness equal to or higher than the threshold value is maintained for a predetermined period of time or longer, or a condition that the stiffness is equal to or higher than the threshold value in a predetermined area or larger, on the basis of the elasticity information. However, any one of these conditions that satisfies a plurality of conditions may be set as a predetermined condition. Specifically, the region extraction unit 36 extracts, from the ultrasonic image, a high stiffness region having stiffness equal to or higher than a predetermined threshold value based on the elasticity information of the observation target in each position. In addition, the region extraction unit 36 may extract a closed region where a relatively stiff region occupies a predetermined area or larger continuously for a predetermined period of time or longer on the basis of the elasticity information calculated by the elasticity information calculation unit 35. In this configuration, it is possible to prevent the number of extracted regions from excessively increasing or prevent a region that is not an actually high stiffness region from being extracted due to noise or the like. The region extraction unit 36 includes a CPU, various operation circuits, or the like.

The calculation unit 37 calculates diagnosis support information that supports an operator to determine a diagnosis sequence of each region on the basis of the elasticity information of each region extracted by the region extraction unit 36. The diagnosis support information includes a priority for diagnosing each region determined by the calculation unit 37, for example, on the basis of the elasticity information of each region. Specifically, the calculation unit 37 calculates an average value of stiffness based on elasticity information of each position included in each region extracted by the region extraction unit 36 and sets the priority in a descending order of this average value. However, the calculation unit 37 may calculate a statistical value of the elasticity information of each region extracted by the region extraction unit 36 (such as the highest value, the most frequent value, and the median value) and sets the priority in a descending order of the statistical value. The calculation unit 37 includes a CPU, various operation circuits, or the like.

The image synthesizing unit 38 creates an image by synthesizing each region extracted by the region extraction unit 36 into an ultrasonic image such that interference with the ultrasonic image is reduced. Specifically, the image synthesizing unit 38 creates an image by synthesizing each region extracted by the region extraction unit 36 into the ultrasonic image so as to be distinguishable using dashed lines, dotted lines, solid lines, or the like. In addition, the image synthesizing unit 38 synthesizes an image by synthesizing the diagnosis support information calculated by the calculation unit 37 into the ultrasonic image created by the image processing unit 33. Specifically, the image synthesizing unit 38 creates an image by synthesizing the priority determined by the calculation unit 37 into the ultrasonic image using a numerical value. In addition, the image synthesizing unit 38 creates an image by synthesizing the elastographic image data of the region of interest into the ultrasonic image created by the image processing unit 33. The image synthesizing unit 38 includes a CPU, various operation circuits, or the like.

The region-of-interest setting unit 39 switches the region of interest sequentially from a region including the high priority region calculated by the calculation unit 37 in response to an input received by the input unit 40. Specifically, the region-of-interest setting unit 39 sets a region including the highest priority region calculated by the calculation unit 37 as the region of interest in response to the input received by the input unit 40. In addition, the region-of-interest setting unit 39 switches the region of interest from the highest priority region to a region including the second highest region in response to the input received by the input unit 40. Then, the region-of-interest setting unit 39 switches the region of interest to a region including the lower priority region in response to the input received by the input unit 40. In this case, the region-of-interest setting unit 39 sets the region of interest by centering a center of mass of the region extracted by the region extraction unit 36 such that a ratio between an area of the extracted region and an area of its surrounding region becomes a predetermined value (for example, 1 : 1).

Note that, in a case where other regions are included when the region-of-interest setting unit 39 sets the region of interest by centering a center of mass of a certain region, the region of interest may be narrowed so as not to include the other regions. In addition, in a case where other regions are included when the region-of-interest setting unit 39 sets the region of interest by centering a center of mass of a certain region, the region of interest may be set so as to include both the regions. In addition, in a case where the region of interest protrudes from the ultrasonic image when the region-of-interest setting unit 39 sets the region of interest by centering a center of mass of a certain region, the region of interest may be narrowed so as not to protrude from the ultrasonic image.

In addition, the region-of-interest setting unit 39 also has a function of setting a region input by an operator using the input unit 40 as the region of interest. The region-of-interest setting unit 39 includes a CPU, various operation circuits, or the like.

The input unit 40 includes an operator interface such as a keyboard, a mouse, a trackball, and a touch panel and receives an input of various types of information. The input unit 40 outputs the received information to the control unit 42. The input unit 40 receives an input from an operator to set the region of interest to a desired region. In addition, the input unit 40 receives an instruction input for switching the region of interest to a region including the lower priority region from an operator.

The storage unit 41 stores data such as various programs for operating the ultrasonic diagnosis system 1 and various parameters or the like necessary for operations of the ultrasonic diagnosis system 1.

In addition, the storage unit 41 stores various programs including an operation program for executing an operation method of the ultrasonic diagnosis system 1. The operation program may be recorded on a computer readable recording medium such as a hard disk, a flash memory, a CD-ROM, a DVD-ROM, and a flexible disk, and may be distributed widely. Note that various programs described above may also be obtained by downloading via a communication network. Here, the communication network includes, for example, an existing public network, a local area network (LAN), a wide area network (WAN), and the like, and it does not matter whether it is wired or wireless.

The storage unit 41 having the aforementioned configuration includes a read-only memory (ROM) where various programs are installed in advance, a random access memory (RAM) where operation parameters or data of each processing are stored, or the like.

The control unit 42 controls the entire ultrasonic diagnosis system 1. The control unit 42 includes a CPU, various operation circuits, or the like having operation and control functions. The control unit 42 reads the information memorized or stored in the storage unit 41 from the storage unit 41 and executes various operation processings relating to an operation method of the ultrasonic observation apparatus 3 to comprehensively control the ultrasonic observation apparatus 3. Note that the control unit 42 may include a CPU or the like commonly used by the signal processing unit 32, the image processing unit 33, the elasticity information calculation unit 35, the region extraction unit 36, the calculation unit 37, the image synthesizing unit 38, and the region-of-interest setting unit 39.

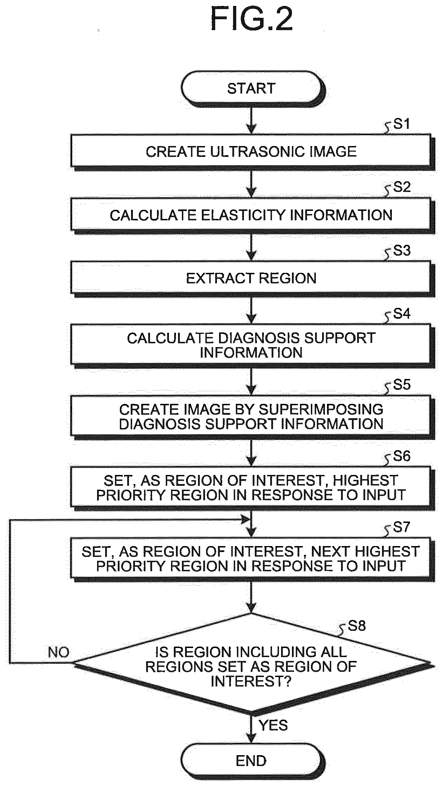

FIG. 2 is a flowchart illustrating an outline of a processing of the ultrasonic observation apparatus according to an embodiment of the invention. First, the image processing unit 33 creates an ultrasonic image of the B-mode image on the basis of the B-mode receive data received from the signal processing unit 32 (Step S1).

In addition, the elasticity information calculation unit 35 calculates elasticity information representing stiffness of each position in the ultrasonic image on the basis of the elastographic receive data received from the signal processing unit 32 (Step S2).

Subsequently, the region extraction unit 36 extracts, from the ultrasonic image, a region having stiffness equal to or higher than a predetermined threshold value based on the elasticity information of the observation target of each position (Step S3).

Then, the calculation unit 37 calculates diagnosis support information that supports an operator to determine a diagnosis sequence of each region on the basis of the elasticity information of each region extracted by the region extraction unit 36 (Step S4). Specifically, the calculation unit 37 determines priorities of each region in a descending order of the high average value of stiffness based on the elasticity information of each region as the diagnosis support information.

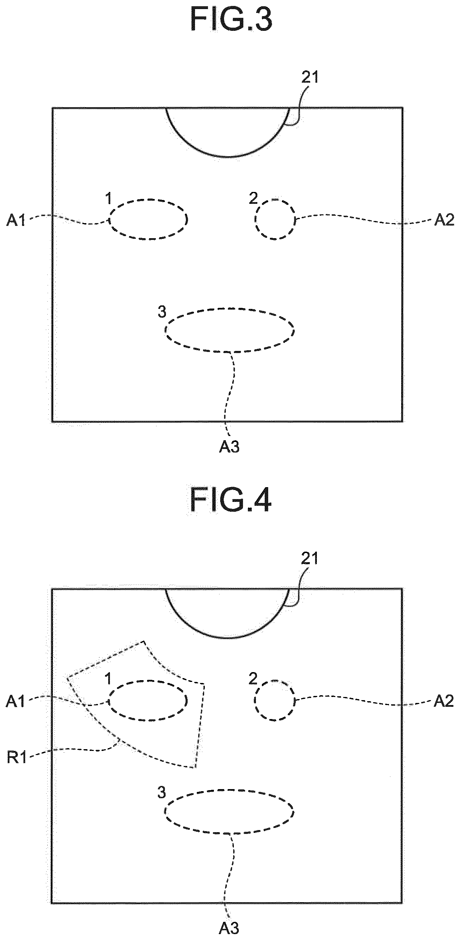

Then, the image synthesizing unit 38 creates an image by synthesizing the priority calculated by the calculation unit 37 into the ultrasonic image created by the image processing unit 33 using a numerical value (Step S5). In addition, the image synthesizing unit 38 creates an image by synthesizing the region extracted by the region extraction unit 36 into the ultrasonic image created by the image processing unit 33 using a dashed line. FIG. 3 is a diagram illustrating an exemplary image displayed on the display device. As illustrated in FIG. 3, the display device 4 displays the image created by the image synthesizing unit 38. Specifically, in the display device 4, regions A1, A2, and A3 extracted by the region extraction unit 36 are displayed as dashed lines, and the priorities of each of the regions A1, A2, and A3 are displayed as numerical values.

Then, as the input unit 40 receives a predetermined input from an operator, the region-of-interest setting unit 39 sets, as the region of interest, a region including the highest priority region calculated by the calculation unit 37 (Step S6). FIG. 4 is a diagram illustrating a state in which a region including the highest priority region is set as the region of interest. As illustrated in FIG. 4, the region-of-interest setting unit 39 sets a region R1 including the highest priority region A1 as the region of interest. Then, the image processing unit 33 creates elastographic image data in the region R1. In addition, the image synthesizing unit 38 creates an image by synthesizing the elastographic image data into the ultrasonic image and displays it on the display device 4.

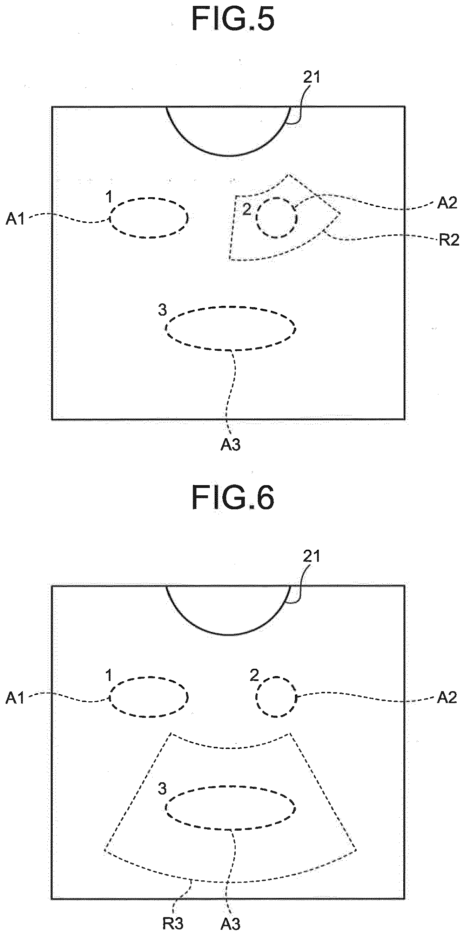

In addition, as the input unit 40 receives a predetermined input from an operator, the region-of-interest setting unit 39 switches the region of interest to a region including the next highest priority region (Step S7). FIG. 5 is a diagram illustrating a state in which a region including the second highest priority region is set as the region of interest. As illustrated in FIG. 5, the region-of-interest setting unit 39 sets a region R2 including the second highest priority region A2 as the region of interest. Then, the image processing unit 33 creates elastographic image data in the region R2. In addition, the image synthesizing unit 38 creates an image by synthesizing the elastographic image data into the ultrasonic image and displays it on the display device 4.

Then, the control unit 42 determines whether or not a region including all the regions extracted by the region extraction unit 36 is set as the region of interest (Step S8). If all the regions are not set as the region of interest (Step S8: No), the process returns to Step S7.

Here, if the input unit 40 receives a predetermined input from an operator, the region-of-interest setting unit 39 switches the region of interest to a region including the next highest priority region (Step S7). FIG. 6 is a diagram illustrating a state in which the region including the third highest priority region is set as the region of interest. As illustrated in FIG. 6, the region-of-interest setting unit 39 sets a region R3 including the third highest priority region A3 as the region of interest. Then, the image processing unit 33 creates elastographic image data in the region R3. In addition, the image synthesizing unit 38 creates an image by synthesizing the elastographic image data into the ultrasonic image and displays it on the display device 4.

Then, the control unit 42 determines whether or not a region including all the regions extracted by the region extraction unit 36 is set as the region of interest (Step S8). If the region including all the regions extracted by the region extraction unit 36 is set as the region of interest (Step S8: Yes), a series of processings is terminated.

As described above, according to the embodiment, an operator can extract a region to be preferentially diagnosed without consuming time or labor. In addition, according to the embodiment, an operator can diagnose a region having the high priority without consuming time or labor. Furthermore, according to the embodiment, an operator can set each extracted region as the region of interest without consuming time or labor.

### (Modifications)

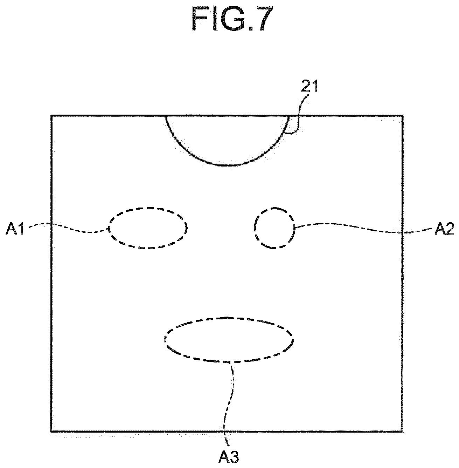

FIG. 7 is a diagram illustrating an exemplary image displayed on the display device in the ultrasonic diagnosis system having the ultrasonic observation apparatus according to a modification of the embodiment. As illustrated in FIG. 7, the image synthesizing unit 38 may visualize the priority determined by the calculation unit 37 using a type of the line surrounding each of the regions A1, A2, and A3 or a color. Specifically, the priority may increase as the line surrounding each of the regions A1, A2, and A3 is closer to red. In addition, the priority may decrease as the line surrounding each of the regions A1, A2, and A3 is closer to blue.

Note that, although a configuration in which the region-of-interest setting unit 39 switches the region of interest sequentially from the region including the high priority region calculated by the calculation unit 37 in response to the input received by the input unit 40 has been described in the aforementioned embodiment, the invention is not limited thereto. For example, after the image illustrated in FIG. 3 is displayed on the display device 4 in Step S5, the region-of-interest setting unit 39 may set, as the region of interest, the region including the region selected by an operator in response to the input received by the input unit 40.

In addition, although a configuration in which the priority is displayed as a numerical value as the diagnosis support information has been described in the aforementioned embodiment, the invention is not limited thereto. For example, an index of the stiffness may be displayed as a numerical value as the diagnosis support information, or a rank depending on the stiffness may be displayed.

In addition, although the calculation unit 37 determines the priority on the basis of the statistical value of stiffness of each region in the aforementioned embodiment, the invention is not limited thereto. For example, the calculation unit 37 may determine the priority in a descending order of the larger area of the region. In addition, an operator may be allowed to select the priority determination method.

In addition, although a configuration in which the image synthesizing unit 38 creates an image by synthesizing each region extracted by the region extraction unit 36 into the ultrasonic image using dashed lines or the like has been described in the aforementioned embodiment, the invention is not limited thereto. For example, the image synthesizing unit 38 may create an image by synthesizing a light color having transmittance in each region extracted by the region extraction unit 36 into the ultrasonic image.

In addition, although a configuration in which the region-of-interest setting unit 39 sets the region of interest by centering a center of mass of the region extracted by the region extraction unit 36 such that a ratio between an area of the extracted region and an area of its surrounding region becomes a predetermined value has been described in the aforementioned embodiment, the invention is not limited thereto. For example, the region-of-interest setting unit 39 may set the region of interest so as to circumscribe the region extracted by the region extraction unit 36.

Further effects or modifications can be easily derived by those skilled in the art. Therefore, the broader aspect of the invention is not limited to the specific details and representative embodiments shown and described above. Accordingly, various modifications may be possible without departing from the spirit or scope of the general inventive concept as defined by the appended claims and their equivalents.

### Reference Signs List

- 1: ULTRASONIC DIAGNOSIS SYSTEM
- 2: ULTRASONIC ENDOSCOPE
- 3: ULTRASONIC OBSERVATION APPARATUS
- 4: DISPLAY DEVICE
- 21: ULTRASONIC TRANSDUCER
- 31: TRANSCEIVER UNIT
- 32: SIGNAL PROCESSING UNIT
- 33: IMAGE PROCESSING UNIT
- 34: FRAME MEMORY
- 35: ELASTICITY INFORMATION CALCULATION UNIT
- 36: REGION EXTRACTION UNIT
- 37: CALCULATION UNIT
- 38: IMAGE SYNTHESIZING UNIT
- 39: REGION-OF-INTEREST SETTING UNIT
- 40: INPUT UNIT
- 41: STORAGE UNIT
- 42: CONTROL UNIT

## Claims

1. An ultrasonic observation apparatus comprising:
an image processing unit configured to create an ultrasonic image based on an ultrasonic signal received from an ultrasonic transducer that transmits an ultrasonic wave to an observation target and receives an ultrasonic wave reflected from the observation target;
an elasticity information calculation unit configured to calculate elasticity information of the observation target in a predetermined region within the ultrasonic image;
a region extraction unit configured to extract, from the predetermined region, a region where the elasticity information calculated by the elasticity information calculation unit satisfies a predetermined condition;
a calculation unit configured to calculate diagnosis support information that supports an operator to determine a diagnosis sequence of each region extracted by the region extraction unit on the basis of the elasticity information of the corresponding region; and
an image synthesizing unit configured to create an image by synthesizing the diagnosis support information calculated by the calculation unit into the ultrasonic image.

2. The ultrasonic observation apparatus according to claim 1, wherein the predetermined condition on the elasticity information includes a condition that stiffness is equal to or higher than a predetermined threshold value, a condition that the stiffness equal to or higher than the threshold value is maintained for a predetermined period of time or longer, or a condition that a region of the stiffness equal to or higher than the threshold value occupies a predetermined area or larger.

3. The ultrasonic observation apparatus according to claim 1 or 2, wherein the diagnosis support information is a priority for diagnosing each region extracted by the region extraction unit, the priority being determined by the calculation unit based on the elasticity information.

4. The ultrasonic observation apparatus according to claim 3, further comprising a region-of-interest setting unit configured to set, as a region of interest, a region including the region extracted by the region extraction unit,
wherein the image synthesizing unit creates an image by synthesizing elastographic image data of the region of interest set by the region-of-interest setting unit into the ultrasonic image.

5. The ultrasonic observation apparatus according to claim 4, further comprising an input unit configured to receive an input of an operator,
wherein the region-of-interest setting unit switches, in response to an input received by the input unit, the region of interest sequentially from the region including the high priority region calculated by the calculation unit.

6. The ultrasonic observation apparatus according to claim 4 or 5, wherein the region-of-interest setting unit sets the region of interest by centering a center of mass of the region extracted by the region extraction unit such that a ratio between an area of the extracted region and an area of a surrounding region thereof becomes a predetermined value.

7. The ultrasonic observation apparatus according to any one of claims 1 to 6, wherein the region extraction unit extracts, based on the elasticity information calculated by the elasticity information calculation unit, a closed region where a relatively stiff region occupies a predetermined area or larger for a predetermined period of time or longer.

8. The ultrasonic observation apparatus according to any one of claims 3 to 7, wherein the calculation unit sets, as the priority, a descending order of higher stiffness based on the elasticity information of each region extracted by the region extraction unit.

9. The ultrasonic observation apparatus according to any one of claims 3 to 7, wherein the calculation unit sets, as the priority, a descending order of a larger area of each region extracted by the region extraction unit.

10. The ultrasonic observation apparatus according to any one of claims 1 to 9, wherein the image synthesizing unit creates an image by synthesizing each region extracted by the region extraction unit into the ultrasonic image such that interference with the ultrasonic image is reduced.

11. The ultrasonic observation apparatus according to any one of claims 1 to 10, wherein the image synthesizing unit creates an image by synthesizing each region extracted by the region extraction unit such that each extracted region is identified with a dashed line, a dotted line, or a solid line.

12. The ultrasonic observation apparatus according to any one of claims 3 to 11, wherein the image synthesizing unit creates an image by synthesizing the priority calculated by the calculation unit such that the priority is identified with a numerical value or a color.

13. A method of operating an ultrasonic observation apparatus that creates an ultrasonic image based on an ultrasonic signal received from an ultrasonic transducer that transmits an ultrasonic wave to an observation target and receives an ultrasonic wave reflected from the observation target, the method comprising:
an elasticity information calculation step of calculating elasticity information of the observation target in a predetermined region within the ultrasonic image using an elasticity information calculation unit;
a region extraction step of extracting, from the predetermined region, a region where the elasticity information calculated by the elasticity information calculation unit satisfies a predetermined condition using a region extraction unit;
a calculation step of calculating diagnosis support information that supports an operator to determine a diagnosis sequence of each region on the basis of the elasticity information of each region extracted by the region extraction unit using a calculation unit; and
an image synthesizing step of creating an image by synthesizing the diagnosis support information calculated by the calculation unit into the ultrasonic image using an image synthesizing unit.

14. A program for operating an ultrasonic observation apparatus that creates an ultrasonic image based on an ultrasonic signal received from an ultrasonic transducer that transmits an ultrasonic wave to an observation target and receives an ultrasonic wave reflected from the observation target, the program causes the ultrasonic observation apparatus to execute steps comprising:
an elasticity information calculation step of calculating elasticity information of the observation target in a predetermined region within the ultrasonic image using an elasticity information calculation unit;
a region extraction step of extracting, from the predetermined region, a region where the elasticity information calculated by the elasticity information calculation unit satisfies a predetermined condition using a region extraction unit;
a calculation step of calculating diagnosis support information that supports an operator to determine a diagnosis sequence of each region on the basis of the elasticity information of each region extracted by the region extraction unit using a calculation unit; and
an image synthesizing step of creating an image by synthesizing the diagnosis support information calculated by the calculation unit into the ultrasonic image using an image synthesizing unit.
